# EUROPEAN PATENT APPLICATION

(11) **EP 4 741 385 A1**
(43) Date of publication of application: **13.05.2026**
(21) Application number: 24836198.2
(22) Date of filing: 28.05.2024
(51) Int. Cl.: C07D 317/38

(54) **METHOD FOR SYNTHESIZING CYCLIC CARBONATE**

(30) Priority: 03.07.2023 KR 20230086066
(71) Applicant: Seoul National University R&DB Foundation, Seoul 08826 (KR)
(72) Inventor: NAM, Ki-Tae, Seoul 06294 (KR); JO, Young-In, Incheon 22202 (KR); JANG, Jun-Ho, Seoul 07220 (KR)
(74) Representative: Maiwald GmbH
(86) International application number: PCT/KR2024/007200
(87) International publication number: WO 2025/009749

(57) **Abstract**

The present invention relates to a method for synthesizing a cyclic carbonate, and more specifically to a method for synthesizing a cyclic carbonate, comprising a step for (i) mixing a compound of chemical formula 1 represented by chemical formula 1 below with a hydrogen carbonate (MHCO₃), or (ii) mixing the compound of chemical formula 1 with a carbonate (M₂CO₃) under acidic conditions. According to the method for synthesizing of the present invention, the use of carbon dioxide, ethylene oxide, propylene oxide, and the like is unnecessary, and a cyclic carbonate having various substituents may be obtained at a high yield even under atmospheric pressure at a relatively low temperature.

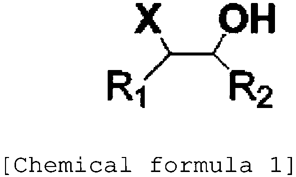

## Description

### Technical Field

The present disclosure relates to a method for synthesizing a cyclic carbonate, and more particularly, to a method for synthesizing a cyclic carbonate for converting a cyclic carbonate substituted with various substituents into a cyclic carbonate in high yield and high concentration without the addition of additional solvents, catalysts, and carbon dioxide, without the use of ethylene oxide, propylene oxide, or the like.

### Background Art

Ethylene carbonate and propylene carbonate are used not only as raw materials for polycarbonate, intermediates for pharmaceuticals, oxyalkylation agents in dye synthesis processes, process equipment protectors, and solvents in fiber production processes, but the scope of use thereof is also expanding recently, including as solvents for polymer electrolytes in secondary batteries.

Synthesis of ethylene carbonate or propylene carbonate known in the prior art was performed by reacting carbon dioxide with ethylene oxide or propylene oxide in the presence of a catalyst, but the accompanying reaction requires high-temperature and high-pressure carbon dioxide gas or liquid to obtain an industrially useful reaction rate. In addition, in the case of ethylene oxide, in the process of synthesizing ethylene oxide from ethylene, there is a problem that a large amount of carbon dioxide is emitted. Since ethylene oxide or propylene oxide tend to be decomposed or polymerized under high-temperature and high-pressure conditions, various catalysts have been developed to alleviate the reaction conditions.

For example, Japanese Patent Laid-Open No. Hei9-67365 discloses a method using Kl (potassium iodide) as a cat alyst, and Japanese Patent Laid-Open No. Sho59-13776 discloses a method using a tetraalkyl phosphonium halide such as tributylmethyl phosphonium iodide as a catalyst. These patents describe that a yield of 50 to 95% is obtained when reacted at a temperature of 100 to 170°C for 1 to 5 hours, but there may be a problem that, to obtain a high yield, the reaction should be performed at a high temperature for a long period of time, and a moisture content of raw materials, carbon dioxide and alkylene oxide, should be controlled to several hundred ppm or less. Thus, a method using an ion exchange resin has been proposed, but this method has the problem of low yield at 80 to 100°C.

In summary, the prior art for commercially preparing various cyclic carbonates require a reaction of gaseous and liquid carbon dioxide at high temperatures and high pressures, and the moisture content of the raw materials should be very low, making the reaction conditions difficult. In addition, despite the development of various catalysts, the generation of carbon dioxide during the process of producing ethylene oxide is unavoidable.

Accordingly, when a technology is developed that does not require the use of high-pressure carbon dioxide, solvents, and catalysts, and does not require the use of ethylene oxide or propylene oxide, and can obtain various cyclic carbonates as a result in high concentrations, it is expected to be widely applied in related fields.

### Summary of Invention

### Technical Problem

An aspect of the present disclosure is to provide a method for synthesizing a cyclic carbonate substituted with various substituents without the need for the use of ethylene oxide and propylene oxide, carbon dioxide, solvents, catalysts, and the like.

### Solution to Problem

Accordingly, according to an aspect of the present disclosure, a method for synthesizing a cyclic carbonate is provided, the method including (i) mixing a compound of chemical formula 1, represented by chemical formula 1 below, with hydrogen carbonate (MHCO₃), or (ii) mixing the compound of chemical formula 1 with carbonate (M₂CO₃) under acidic conditions.

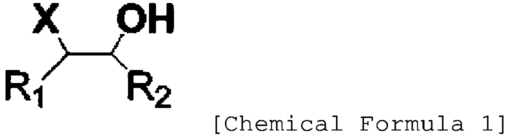

In chemical formula 1 above, X is a halogen selected from the group consisting of fluorine, chlorine, bromine, and iodine,
R₁ is hydrogen,
R₂ is independently selected from the group consisting of a vinyl group, a hydroxyl group, and a halogen group selected from the group consisting of fluorine, chlorine, bromine, and iodine, and
M is a cation (M⁺) selected from the group consisting of an alkali metal cation, an ammonium ion (NH₄⁺), a primary ammonium ion, a secondary ammonium ion, and a tertiary ammonium ion.

### Advantageous Effects of Invention

According to the present disclosure, according to the method for synthesizing a cyclic carbonate, the use or additional input of ethylene oxide, propylene oxide, and carbon dioxide may not be required, and various cyclic carbonate derivatives may be obtained in high yields even under relatively low temperature and low pressure conditions, and the use of a solvent may be excluded, so a subsequent purification process may be simple, which is preferable in terms of process economy.

### Brief Description of Drawings

FIG. 1 illustrates the results of observing a product present in a reaction solution at 80°C during the synthesis of a cyclic carbonate through an NMR analysis by the process of Preparation Example 1.
FIG. 2 illustrates the results of observing a product present in a reaction solution at 80°C during the synthesis of a cyclic carbonate through an NMR analysis by the process of Preparation Example 2.
FIG. 3 illustrates the results of observing a product present in a reaction solution at 80°C during the synthesis of a cyclic carbonate through an NMR analysis by the process of Preparation Example 3.
FIG. 4 is a graph illustrating a yield of cyclic carbonate derivatives depending on the type of a reactant.
FIG. 5 is a graph illustrating a yield of glycerol carbonate according to a reaction temperature when using 3-bromo-1,2-propanediol as a reactant.
FIG. 6 is a graph illustrating a yield of 4-bromomethyl-1,3-dioxolan-2-one according to a reaction temperature when using 1,3-dibromo-2-propanol as a reactant.
FIG. 7 is a graph illustrating a yield of 4-bromomethyl-1,3-dioxolan-2-one according to a bicarbonate cation when using 1,3-dibromo-2-propanol as a reactant.
FIG. 8 is a graph illustrating yields of 4-bromomethyl-1,3-dioxolan-2-one and glycerol carbonate when carbonate and acid are introduced when using 1,3-dibromo-2-propanol as a reactant.

### Best Mode for Invention

Hereinafter, preferred embodiments of the present disclosure will be described with reference to the attached drawings. However, the embodiments of the present disclosure may be modified in various other forms, and the scope of the present disclosure is not limited to the embodiments described below.

In a method for synthesizing a cyclic carbonate according to the present disclosure, the use of ethylene oxide, propylene oxide, carbon dioxide, or the like, may not be required, and a cyclic carbonate may be obtained in high yield even under relatively low temperature and low pressure conditions, and the use of a solvent may be excluded, so a subsequent purification process may be simple, which is preferable in terms of process economy.

In addition, according to the method for synthesizing a cyclic carbonate of the present disclosure, a cyclic carbonate derivative having various substituents may be synthesized.

More specifically, the method for synthesizing a cyclic carbonate of the present disclosure includes an operation of (i) mixing a compound of chemical formula 1, represented by chemical formula 1 below, with hydrogen carbonate (MHCO₃), or (ii) mixing the compound of chemical formula 1 with carbonate (M₂CO₃) under acidic conditions.

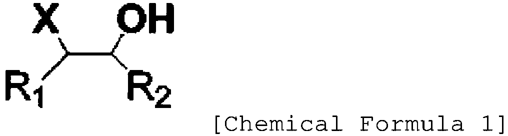

In this case, in chemical formula 1 above, X is a halogen selected from the group consisting of fluorine, chlorine, bromine, and iodine, R₁ is hydrogen, R₂ is independently selected from the group consisting of a halogen selected from the group consisting of fluorine, chlorine, bromine, and iodine; a hydroxy group; and a vinyl group, M is a component corresponding to a cation selected from the group consisting of an alkali metal cation, an ammonium ion (NH₄⁺), a primary ammonium ion, a secondary ammonium ion, and a tertiary ammonium ion, and may be selected from the group consisting of an alkali metal, ammonium, a primary ammonium, a secondary ammonium, and a tertiary ammonium, and when expressed in ionic form, M may be expressed as "M⁺", and therefore M and M⁺ may be used interchangeably depending on the state.

More specifically, the cation (M⁺) may be an alkali metal cation, for example, selected from the group consisting of Li, Na, K and Cs. In addition, the cation (M⁺) may be an organic nitrogen compound-based cation, for example, a cation selected from the group consisting of an ammonium ion (NH₄⁺) , primary ammonium ions (NH₃⁺CH₂CH₂OH, NH₃⁺CH₂CH₃, NH₃⁺CH₂CH₂CH₃, or the like), secondary ammonium ions (NH₂⁺(CH₂CH₂OH)₂), NH₂⁺(CH₂CH₃)₂, NH₂⁺(CH₂CH₂CH₃)₂, or the like), and tertiary ammonium ions (NH⁺(CH₂CH₂OH)₃), NH⁺(CH₂CH₃)₃, NH⁺(CH₂CH₂CH₃)₃, or the like).

In the compound of chemical formula 1, positions of X and -OH groups may be exchanged, and positions of R1 and R2 may also be exchanged.

More specifically, the compound represented by chemical formula 1 above may be one of compounds of chemical formulas 1-1, 1-2 and 1-3 below.

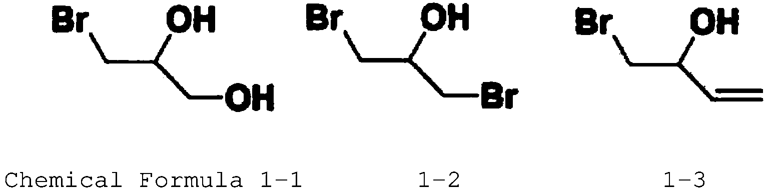

For example, when 3-Bromo-1,2-propanediol is used as the compound of chemical formula 1-1, the synthesized cyclic carbonate may be glycerol carbonate, which is a compound of chemical formula 2-1 below, when 1,3-Dibromo-2-propanol is used as the compound of chemical formula 1-2, the synthesized cyclic carbonate may be 4-bromomethyl-1,3-dioxolan-2-one, which is a compound of chemical formula 2-2 below, and when 1-Bromo-3-buten-2-ol is used as the compound of chemical formula 1-3, the synthesized cyclic carbonate may be vinyl ethylene carbonate, which is a compound of chemical formula 2-3 below.

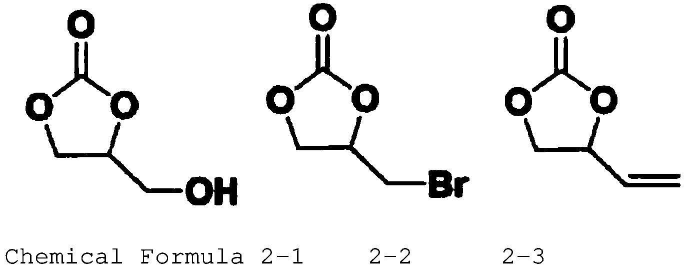

The compound of chemical formula 1, which is a reactant of the present disclosure, may be in a liquid state.

The hydrogen carbonate (MHCO₃) used in the method for synthesizing a cyclic carbonate of the present disclosure may be in a liquid state or a solid state. More specifically, when the method for synthesizing a cyclic carbonate of the present disclosure includes the mixing operation of (i), a liquid compound of chemical formula 1 and a solution or solid hydrogen carbonate (MIHCO₃) are used as reactants. However, even in this case, according to the present disclosure, ethylene oxide, propylene oxide, additional carbon dioxide, solvent, or the like, are not required. The hydrogen carbonate (MHCO₃) is preferably in the form of solid powder.

By performing the mixing operation, a cyclic carbonate may be obtained, and since a solvent is not essential according to the present disclosure, a subsequent separation process of the solvent from a product is simplified, so that a cyclic carbonate in high concentration may be obtained as a product in a single operation.

For example, when a bicarbonate (HCO₃⁻) derived from a hydrogen carbonate in a liquid state or a solid state reacts with the liquid compound of chemical formula 1, MX among the reaction products may be obtained in a solid phase and therefore may be easily separated by solid-liquid separation.

Meanwhile, when the method for synthesizing a cyclic carbonate of the present disclosure includes the mixing operation of (ii), the liquid compound of chemical formula 1 is mixed with a solution or solid carbonate (M₂CO₃), and a solution or liquid acid as reactants. However, in this case, the mixing operation of the compound of chemical formula 1, carbonate, and acid is not particularly limited, and the compound of formula 1 and carbonate may be mixed under acidic conditions. The carbonate (M₂CO₃) is preferably in the form of solid powder.

The acidic conditions of the mixing operation of (ii) may be mixing an acid in the range of 0.5 equivalents to 1.5 equivalents, relative to carbonate (M₂CO₃), and preferably mixing an acid in the range of 0.5 equivalents to 1 equivalent, relative to carbonate (M₂CO₃). There is a problem that a reaction yield may be decreased in the case that the acidic conditions are below the equivalent range, and there is also a problem that the reaction yield may be decreased when the acidic conditions are above the equivalent range.

For example, acid that can be used in the acidic conditions may be at least one acid selected from the group consisting of organic acids having a carboxyl group, such as nitric acid, hydrochloric acid, bromic acid, formic acid, and acetic acid.

The mixing operation of (i) or (ii) may be performed at a temperature of 40°C or higher and lower than 150°C , preferably 80°C to 100°C, and in this case, a cyclic carbonate may be prepared with a yield of 70% or more, or even 80% or more, without applying additional pressure in the mixing operation.

Meanwhile, in the operation of preparing a cyclic carbonate, when the temperature is below the range of the present disclosure, the reaction may become somewhat slow, and when the temperature exceeds the range of the present disclosure, the following side reactions may increase, thereby lowering the yield of the cyclic carbonate.

The time at which the mixing operation of (i) or (ii) of the present disclosure is performed may be from 15 minutes to 24 hours, for example from 30 minutes to 6 hours. When the reaction time is less than 15 minutes, a yield of a product may be insufficient, and when the reaction time exceeds 24 hours, an increase in the yield of the product due to an increase in the reaction time is not significant, which is not preferable in terms of process economy.

The method for synthesizing a cyclic carbonate of the present disclosure may be performed preferably using a closed reactor, wherein external pressure of the reaction vessel may be maintained at atmospheric pressure, and internal pressure of the closed vessel may change as the reaction proceeds. In addition, the reaction may proceed even if both the internal and external pressures are maintained at atmospheric pressure since the vessel is not sealed. Therefore, the mixing operation of (i) or (ii) may be performed under atmospheric pressure conditions.

The mixing operation of (i) may be performed by mixing a compound of chemical formula 1 with hydrogen carbonate (MHCO₃) in a molar ratio of 1:0.5 to 2, and preferably mixing the same in the same molar ratio of 1:1.

The mixing operation of (ii) may be performed by mixing the compound of chemical formula 1 with carbonate (MCO₃) in a molar ratio of 1:0.5 to 1:2, and preferably in a molar ratio of 1:0.5 to 1:1.

Meanwhile, the mixing operation of (ii) may be performed by mixing carbonate (MCO₃) with acid in a molar ratio of 1:0.5 to 1:2, and preferably in a molar ratio of 1:1.

However, the present disclosure does not exclude the use of an additional solvent in the mixing operation of (i) or (ii), and if required, water; various types of polar organic solvents such as alcohol, dimethylformamide, acetone, ether, and ester; and a mixture of at least two or more of the components may be used as solvents. Preferably, water may be mixed as a solvent.

Accordingly, the mixing operation of (i) or (ii) may be performed without a solvent or in the presence of at least one polar organic solvent selected from the group consisting of water; alcohol, dimethylformamide, acetone, ether and ester; or a mixture thereof.

According to the method for synthesizing a cyclic carbonate of the present disclosure, a cyclic carbonate may be obtained in high yield and high concentration, and a subsequent purification process is not required. However, in some cases, a purification operation may be performed to improve purity, and the operation is not excluded. For example, a drying operation may be additionally performed.

As described above, according to the present disclosure, the use of catalyst, solvent, ethylene oxide, propylene oxide, and carbon dioxide at a high temperature and high pressure is not necessarily required, and the process may be performed in a single operation at a mild temperature and mild pressure, and as a result, a cyclic carbonate may be obtained in high yield, and subsequent purification is not essential.

Hereinafter, the present disclosure will be described in more detail through specific Examples. The following examples are merely illustrative examples to aid understanding of the present disclosure, and the scope of the present disclosure is not limited thereto.

### Mode for Invention

### Example

### 1. Synthesis of Cyclic Carbonate Derivatives

### Preparation Example 1

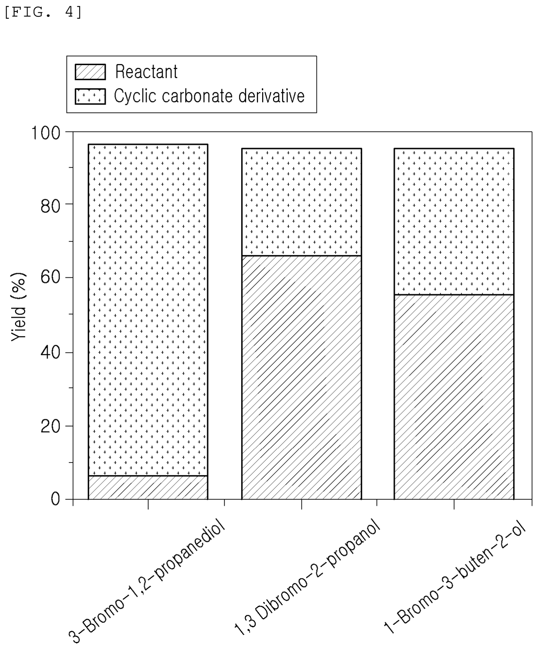

In a hydrothermal synthesis reaction vessel with a capacity of 11 mL, 7.75g of liquid 3-bromo-1,2-propanediol, 9.81g of liquid 1,3-dibromo-2-propanol, or 7.55g of liquid 1-bromo-3-buten-2-ol were mixed with 4.20g, 3.78g, and 4.20g of NaHCO₃ powder, respectively, and placed in the vessel, and then sealed under atmospheric pressure conditions and heated at 80°C for 12 hours, and then a product present in a reaction solution was observed. The results are illustrated in FIG. 4.

### Preparation Example 2

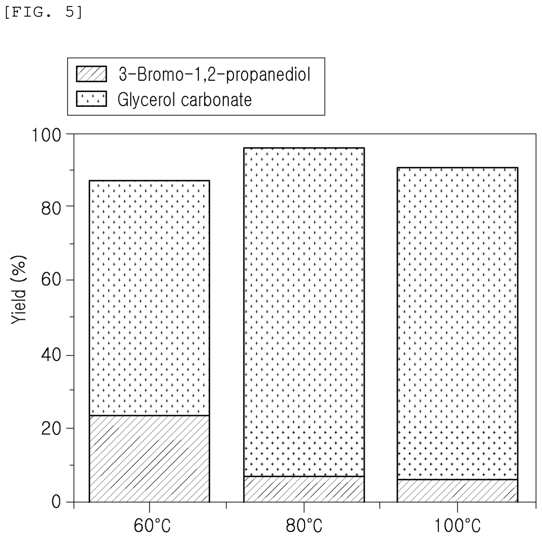

In a hydrothermal synthesis reaction vessel with a capacity of 11 mL, 7.75g of liquid 3-bromo-1,2-propanediol were mixed with 4.20g of NaHCO₃ powder, and placed in the vessel and then sealed under atmospheric pressure conditions and heated at 60°C, 80°C, and 100°C for 12 hours, and then a product present in a reaction solution was observed. The results are illustrated in FIG. 5.

### Preparation Example 3

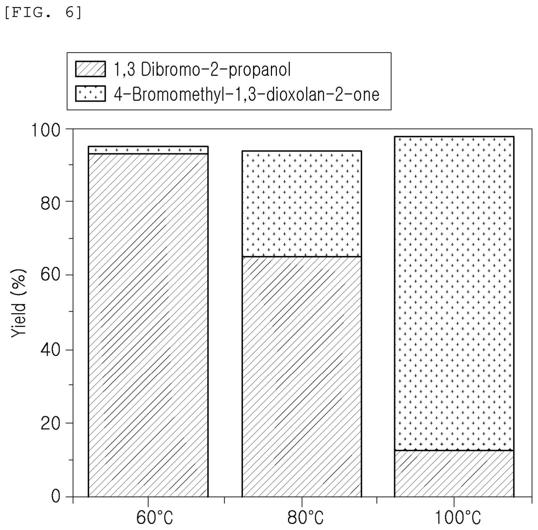

In a hydrothermal synthesis reaction vessel with a capacity of 11 mL, 9.81g of liquid 1,3-dibromo-2-propanol and 3.78g of NaHCO₃ powder, and placed in the vessel and then sealed under atmospheric pressure conditions, and heated at 60°C, 80°C, and 100°C for 12 hours, and then a product present in a reaction solution was observed. The results are illustrated in FIG. 6.

### Preparation Example 4

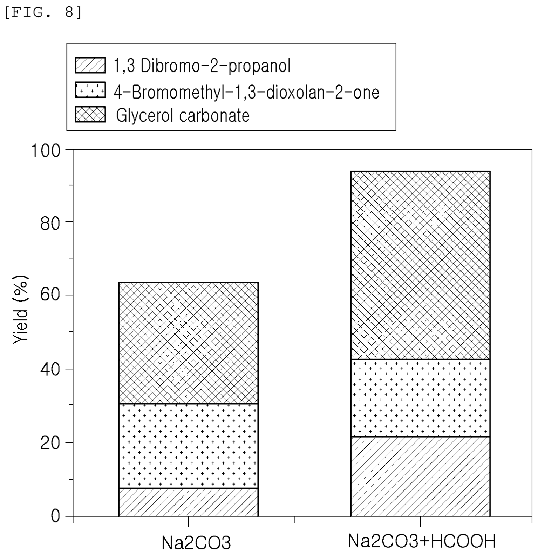

In a hydrothermal synthesis reaction vessel with a capacity of 11 mL, 6.54g of liquid 1,3-dibromo-2-propanol, 3.18g of Na₂CO₃ powder, and 0 mL or 2.26 mL of formic acid were mixed respectively, and placed in the vessel and then sealed under atmospheric pressure, and heated at 100°C for 12 hours, and then a product present in a reaction solution was observed. The results are illustrated in FIG. 8.

### Comparative Preparation Example 1

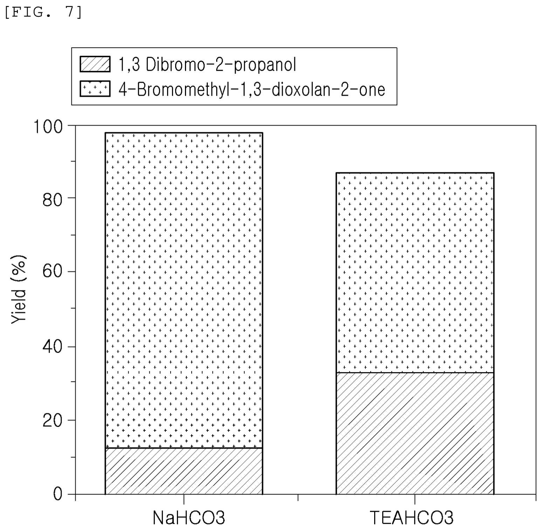

In a hydrothermal synthesis reaction vessel with a capacity of 11 mL, 9.81g of liquid 1,3-dibromo-2-propanol and 3.78g of NaHCO₃ powder, and 5.45g of 1,3-dibromo-2-propanol and 4.89g of tetraethylammonium bicarbonate (TEAHCO₃) powder were mixed respectively, and placed in the vessel and then sealed under atmospheric pressure, and heated at 100°C for 12 hours, and then a product present in a reaction solution was observed. The results are illustrated in FIG. 7.

### 2. Product Confirmation

### (1) Changes in product according to reactant

When a cyclic carbonate was synthesized using the process of Preparation Example 1, a product present in a reaction solution at 80°C was observed through an NMR analysis.

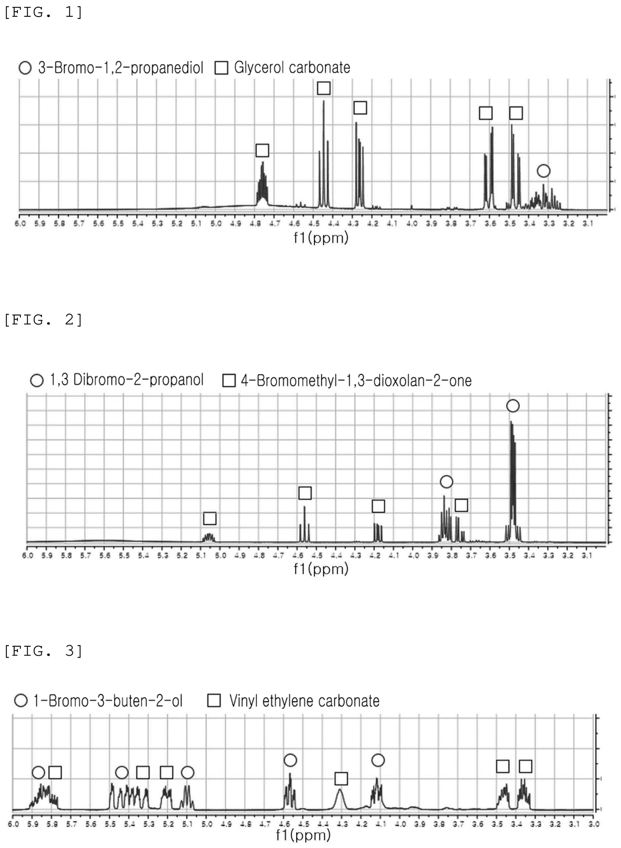

An NMR of the reaction solution after heating was measured, and the results illustrated in FIG. 1 were confirmed.

In addition, a yield of cyclic carbonate was illustrated as in FIG. 4, and it was confirmed that a cyclic carbonate derivative may be synthesized from a brominated alcohol derivative according to the method for synthesizing a cyclic carbonate of the present disclosure.

### (2) Changes in product according to reaction temperature

When cyclic carbonate derivatives were synthesized by the processes of Preparation Examples 2 and 3, products present in a reaction solution at 60°C, 80°C, and 100°C were observed using an NMR.

The NMR of the reaction solution after heating was measured, and the results illustrated in FIGS. 2 and 3 were confirmed.

In addition, as can be seen in FIG. 5, it could be confirmed that glycerol carbonate may be obtained with a yield of 90% or more at a temperature of 80 to 100°C by the method for synthesizing a cyclic carbonate of the present disclosure.

In addition, as can be seen in FIG. 6, it could be confirmed that 4-bromomethyl-1,3-dioxolan-2-one may be obtained with a yield of 86% at a temperature of 100°C.

### (3) Changes in product according to cation of bicarbonate

When a cyclic carbonate derivative was synthesized using the process of Comparative Preparation Example 1, a product present in a reaction solution was observed using an NMR depending on the type of cation of the bicarbonate to a bicarbonate cation after heating at 100°C for 12 hours.

As can be seen in FIG. 7, 4-bromomethyl-1,3-dioxolan-2-one could be obtained with a yield of 86% using NaHCO₃. However, 4-bromomethyl-1,3-dioxolan-2-one could be obtained with a yield of 56% using TEAHCO₃. This is interpreted as being because TEAHCO₃ has a lower solubility in 1,3-dibromo-2-propanol than that of NaHCO₃, and TEABr has a lower ability to absorb water generated after the reaction than that of NaBr.

### (4) Observation of cyclic carbonate production under conditions using carbonate and acid

When a cyclic carbonate derivative was synthesized using the process of Preparation Example 4, a product present in a reaction solution was observed using an NMR after heating at 100°C for 12 hours.

As the result, as can be seen in FIG. 8, when Na₂CO₃ and HCOOH were introduced simultaneously, 4-bromomethyl-1,3-dioxolan-2-one could be obtained with a yield of 21% and glycerol carbonate could be obtained with a yield of 50%. In the case of glycerol carbonate, the 4-bromomethyl-1,3-dioxolan-2-one produced through the reaction is converted into glycerol carbonate through an additional reaction. It was confirmed that cyclic carbonates can be synthesized through the reaction of Na₂CO₃ and acid.

In addition, when using only Na₂CO₃, 4-bromomethyl-1,3-dioxolan-2-one could be obtained with a yield of 22% and glycerol carbonate with a yield of 33%. The lower reactivity compared to that of NaHCO₃ is due to the strong bonding of Na₂CO₃ with CO₂ molecules.

While examples have been described in detail above, the scope of the present disclosure is not limited thereto, and it will be apparent to those skilled in the art that modifications and variations could be made without departing from the technical idea of the present disclosure described in the claims.

## Claims

1. A method for synthesizing a cyclic carbonate, comprising:
(i) mixing a compound of chemical formula 1, represented by chemical formula 1 below, with hydrogen carbonate (MHCO₃), or
(ii) mixing the compound of chemical formula 1 with carbonate (M₂CO₃) under acidic conditions, 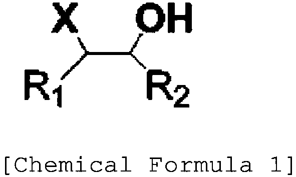 In chemical formula 1 above,
X is a halogen selected from the group consisting of fluorine, chlorine, bromine, and iodine, R₁ is hydrogen,
R₂ is independently selected from the group consisting of a vinyl group, a hydroxyl group, and a halogen selected from the group consisting of fluorine, chlorine, bromine, and iodine, and
M is a cation (M⁺) selected from the group consisting of an alkali metal cation, an ammonium ion (NH₄⁺), a primary ammonium ion, a secondary ammonium ion, and a tertiary ammonium ion.

2. The method for synthesizing a cyclic carbonate of claim 1, wherein the compound represented by chemical formula 1 above is one of compounds represented by chemical formulas 1-1, 1-2, and 1-3 below,

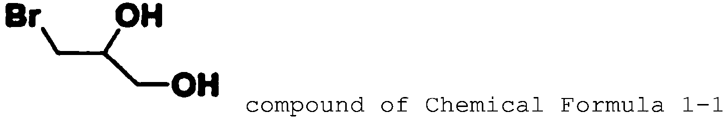

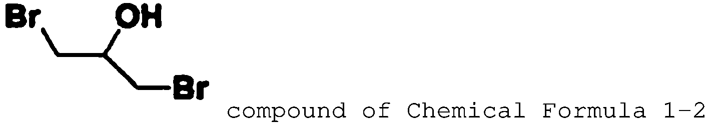

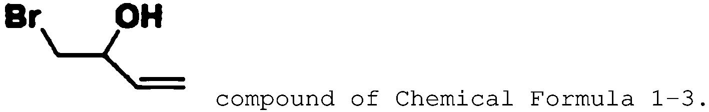

3. The method for synthesizing a cyclic carbonate of claim 1, wherein the compound of chemical formula 1 above is in a liquid state.

4. The method for synthesizing a cyclic carbonate of claim 1, wherein the hydrogen carbonate (MHCO₃) is in a liquid state or in a solid state.

5. The method for synthesizing a cyclic carbonate of claim 1, wherein the acidic conditions are controlled by an acid selected from the group consisting of nitric acid, hydrochloric acid, bromic acid, formic acid, and acetic acid.

6. The method for synthesizing a cyclic carbonate of claim 1, wherein the mixing operation of(i) is performed at a temperature within a range of 40°C or higher and lower than 150°C.

7. The method for synthesizing a cyclic carbonate of claim 1, wherein the mixing operation of (i) is performed by mixing the compound of chemical formula 1 with hydrogen carbonate (MHCO₃) in a molar ratio of 1:0.5 to 2.

8. The method for synthesizing a cyclic carbonate of claim 1, wherein the mixing operation of (i) or(ii) is performed under atmospheric pressure conditions.

9. The method for synthesizing a cyclic carbonate of claim 2, wherein the synthesized cyclic carbonate is one of compounds represented by chemical formulas 2-1, 2-2, and 2-3 below,

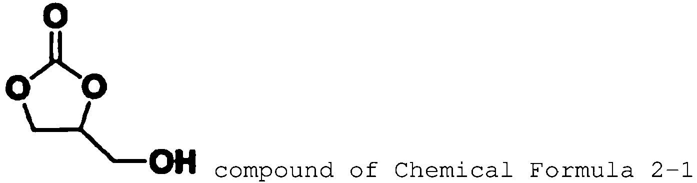

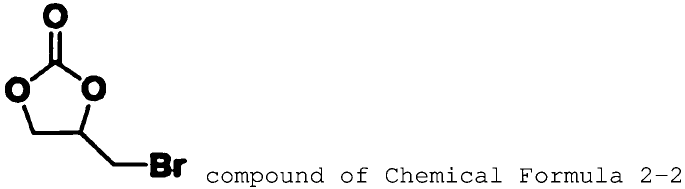

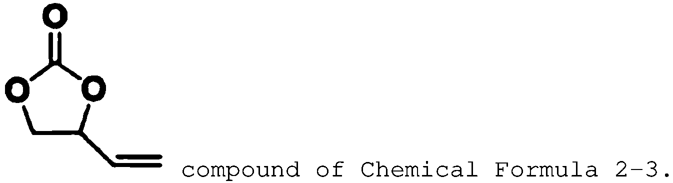

10. The method for synthesizing a cyclic carbonate of claim 1, wherein the mixing operation of (i) or(ii) is performed without a solvent; or
is performed under water; at least one polar organic solvent selected from the group consisting of alcohol, dimethylformamide, acetone, ether and ester; or a mixture thereof.
